# EUROPEAN PATENT APPLICATION

(11) **EP 4 578 415 A1**
(43) Date of publication of application: **02.07.2025**
(21) Application number: 24222715.5
(22) Date of filing: 23.12.2024
(51) Int. Cl.: A61B 18/14

(54) **FLOW DIVERTER FOR A CATHETER**

(30) Priority: 27.12.2023 US 202318397853
(71) Applicant: Biosense Webster (Israel) Ltd., 2066717 Yokneam (IL)
(72) Inventor: MAI, Tung, Irvine, 92618 (US)
(74) Representative: Carpmaels & Ransford LLP

(57) **Abstract**

The disclosed technology includes a flow diverter comprising a lumen extending along a longitudinal axis from a proximal end to a distal end of the flow diverter. The flow diverter can comprise a plurality of manifolds, each manifold comprising a plurality of fluid passageways, each fluid passageway extending from the lumen to an exterior of the flow diverter. The flow diverter can further comprise a plurality of annular ridges disposed around the lumen between the proximal end and the distal end of the flow diverter. Each manifold of the plurality of manifolds can be disposed between adjacent annular ridges of the plurality of annular ridges.

## Description

### FIELD

The present invention relates generally to medical devices, and in particular medical probes with irrigation, and further relates to, but not exclusively, medical probes configured to provide irrigation to electrodes.

### BACKGROUND

Cardiac arrhythmias, such as atrial fibrillation (AF), occur when regions of cardiac tissue abnormally conduct electrical signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art utilize radiofrequency (RF) electrical energy to heat tissue. RF ablation can have certain risks related to thermal heating which can lead to tissue charring, burning, steam pop, phrenic nerve palsy, pulmonary vein stenosis, and esophageal fistula.

Prior to ablating tissue, it may be desirable to map out the pulmonary vein or other anatomic features. During mapping, the chance of inducing blood clots may increase. Further, ablation of tissue can cause localized temperature increases near the electrodes. For at least these reasons, it is desirable to provide an irrigation fluid to the anatomical region being treated or mapped. Unfortunately, many existing irrigation elements are designed with sharp turns or otherwise inefficient designs that can reduce the effectiveness of the cooling provided by the irrigation elements. Accordingly, there is a need in the art for irrigation elements that increase the effectiveness of the cooling provided by the irrigation elements.

### SUMMARY

There is provided, in accordance with an example of the present invention, a flow diverter comprising: a proximal end and a distal end; a lumen extending along a longitudinal axis from the proximal end to the distal end of the flow diverter; a plurality of annular ridges disposed around the lumen between the proximal end and the distal end; and a plurality of manifolds, each manifold can be disposed between adjacent annular ridges of the plurality of annular ridges and comprising a plurality of fluid passageways, and each fluid passageway can extend from the lumen to an exterior of the flow diverter.

The distal end of the flow diverter can comprise a distal aperture, the distal aperture can be configured to form a seal around an outer circumference of an end effector disposed through the lumen. The plurality of annular ridges can comprise a proximal annular ridge, a second annular ridge, a third annular ridge, and a distal annular ridge. The plurality of manifolds can comprise a first manifold disposed between the proximal annular ridge and the second annular ridge, a second manifold disposed between the second annular ridge and the third annular ridge, and a third manifold disposed between the third annular ridge and the distal annular ridge. Each of the proximal annular ridge, the second annular ridge, and the third annular ridge can comprise a distal surface and a proximal surface, and each of the proximal surfaces can be substantially convex and each of the distal surfaces can be substantially concave. The distal annular ridge can comprise a distal surface and a proximal surface, and both the distal surface and the proximal surface of the distal annular ridge can be substantially convex. A diameter of the proximal annular ridge can be greater than a diameter of the second annular ridge, the diameter of the second annular ridge can be greater than a diameter of the third annular ridge, and the diameter of the third annular ridge can be greater than a diameter of the distal annular ridge. The flow diverter can comprise an elastomeric material. The flow diverter can comprise silicone.

The disclosed technology can include a catheter comprising an insertion shaft extending along a longitudinal axis; an end effector disposed at a distal end of the insertion shaft; a sheath disposed around the insertion shaft; and a flow diverter disposed at a distal end of the sheath, the flow diverter comprising; a proximal end and a distal end; a lumen extending along the longitudinal axis from the proximal end to the distal end of the flow diverter; a plurality of annular ridges disposed around the lumen between the proximal end and the distal end; and a plurality of manifolds, and each manifold can be disposed between adjacent annular ridges of the plurality of annular ridges and comprising a plurality of fluid passageways, and each fluid passageway can extend from the lumen to an exterior of the flow diverter.

The plurality of manifolds can divert irrigation fluid from the lumen to the exterior of the flow diverter to distribute the irrigation fluid radially about the end effector. The end effector can comprise one or more electrodes configured for tissue ablation, and the irrigation fluid can cool the electrodes during ablation. The end effector can be a guidewire.

A diameter of each annular ridge of the plurality of annular ridges may decrease relative to one another from the proximal end to the distal of the flow diverter. The plurality of annular ridges can comprise a proximal annular ridge, a second annular ridge, a third annular ridge, and a distal annular ridge. The plurality of manifolds can comprise a first manifold disposed between the proximal annular ridge and the second annular ridge, a second manifold disposed between the second annular ridge and the third annular ridge, and a third manifold disposed between the third annular ridge and the distal annular ridge.

Each of the proximal annular ridge, the second annular ridge, and the third annular ridge can comprise a distal surface and a proximal surface, and each of the proximal surfaces can substantially convex and each of the distal surfaces can be substantially concave. The distal end of the flow diverter can comprise a distal aperture, the distal aperture can form a seal about an outer circumference of the end effector disposed through the lumen. The seal can comprise an elastomeric material. The seal can comprise silicone.

Additional features, functionalities, and applications of the disclosed technology are discussed in more detail herein.

### BRIEF DESCRIPTION OF THE DRAWINGS

FIG. 1 is a schematic pictorial illustration of a medical system including a medical probe, in accordance with the disclosed technology;
FIG. 2 is a schematic pictorial illustration showing a perspective view of a medical probe, in accordance with the disclosed technology;
FIG. 3A is a schematic pictorial illustration showing a perspective view of flow diverter, in accordance with the disclosed technology;
FIG. 3B is a schematic pictorial illustration showing an exploded perspective view of flow diverter, in accordance with the disclosed technology;
FIG. 3C is a schematic pictorial illustration showing an exploded side view of flow diverter, in accordance with the disclosed technology;
FIG. 3D is a schematic pictorial illustration showing a sectional view of flow diverter, in accordance with the disclosed technology;
FIG. 3E is a schematic pictorial illustration showing a sectional view of flow diverter, in accordance with the disclosed technology;
FIG. 4A is a schematic pictorial illustration depicting proximal end components of the flow diverter, in accordance with the disclosed technology; and
FIG. 4B is a schematic pictorial illustration depicting a collar component of the flow diverter, in accordance with the disclosed technology.

### DETAILED DESCRIPTION

The following detailed description should be read with reference to the drawings, in which like elements in different drawings are identically numbered. The drawings, which are not necessarily to scale, depict selected examples and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 71% to 110%. In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "physician" can include a doctor, surgeon, technician, scientist, operator, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as reversible or irreversible electroporation (IRE), referred throughout this disclosure interchangeably as pulsed electric field (PEF) and pulsed field ablation (PFA), or thermal energy such as radiofrequency (RF) ablation or cryoablation. Ablating or ablation as it relates to the devices and corresponding systems of this disclosure is used throughout this disclosure in reference to thermal or non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

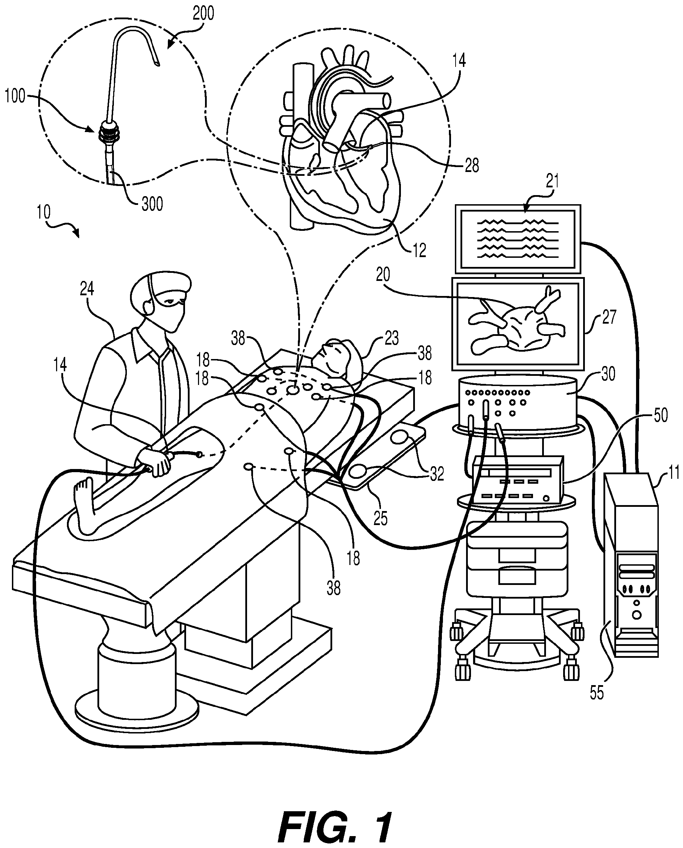

FIG. 1 shows an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. In an example of sensing IEGM signals, Physician 24 brings a distal tip of catheter 14 (i.e., an end effector 200 in this case) to a target site in heart 12. For ablation, physician 24 would similarly bring a distal end of an ablation catheter to a target site for ablating.

Catheter 14 is an exemplary catheter that includes a sheath 300 including a flow diverter 100 disposed at a distal end of the sheath 300. The catheter 14 may further include an end effector 200 which passes through the sheath 300 and flow diverter. The end effector 200 may include one or more electrodes configured to detect electro-physiological signals and/or deliver ablative energy to tissue. Catheter 14 may additionally include a magnetic-based position sensor embedded in or near end effector 200 for tracking position and orientation of end effector 200. The end effector 200 can further include one or more impedance-based electrodes disposed in or near end effector 200 for tracking position and orientation of end effector 200. In some examples, the end effector 200 is a guidewire.

Magnetic-based position sensor may be operated together with a location pad 25 including a plurality of magnetic coils 32 configured to generate magnetic fields in a predefined working volume. Real time position of end effector 200 of catheter 14 may be tracked based on magnetic fields generated with location pad 25 and sensed by magnetic-based position sensor 29. The magnetic-based position sensor can be a single axis sensor, a dual axis sensor, or a triple axis sensor depending on the particular configuration. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; and 6,892,091, each of which is incorporated herein by reference as if set forth fully herein.

System 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which are incorporated herein by reference.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618, USA.

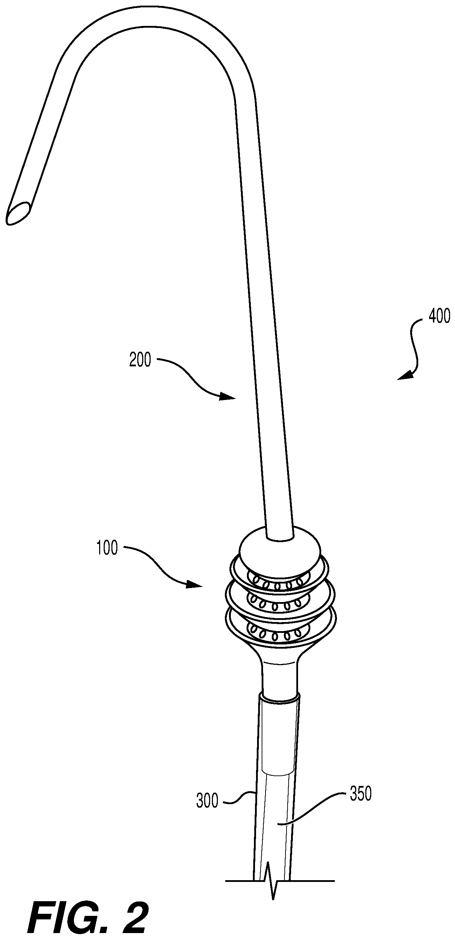

As shown in FIG. 2 the catheter 14 may comprise a sheath 300. A flow diverter 100 may be disposed at a distal end of the sheath 300. An end effector 200 may be disposed at a distal end of an insertion shaft 350. The flow diverter 100 may be configured to deliver irrigation fluid radially about the end effector 200 and to a target anatomical region. The end effector 200 may be provided though the flow diverter 100, and a distal end of the flow diverter 100 may be configured to create a seal around the circumference of the end effector 200. In some examples, the flow diverter 100 can be provided within an interior of a basket shaped catheter to provide irrigation to electrodes of said catheter during operation.

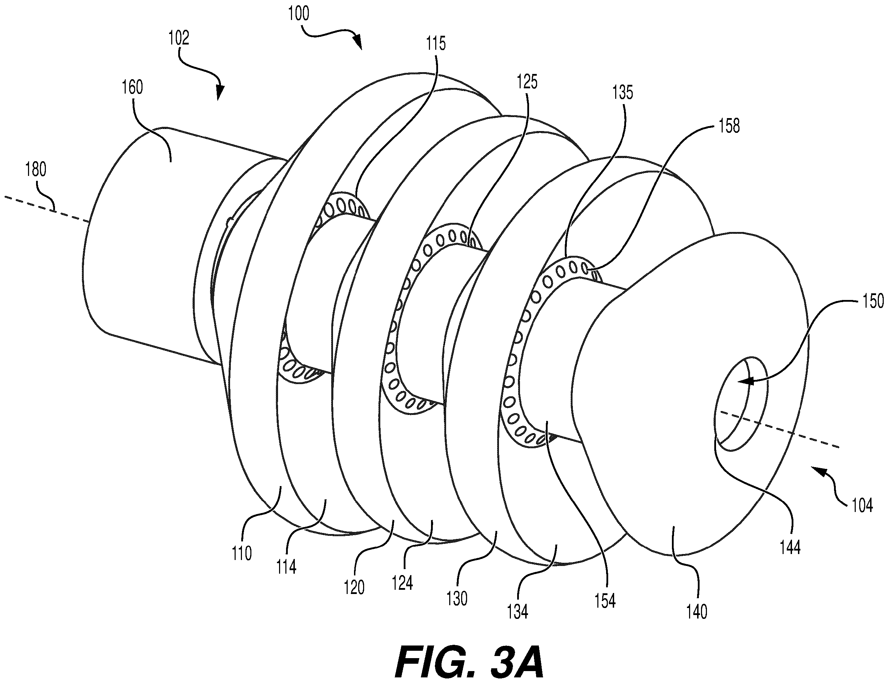

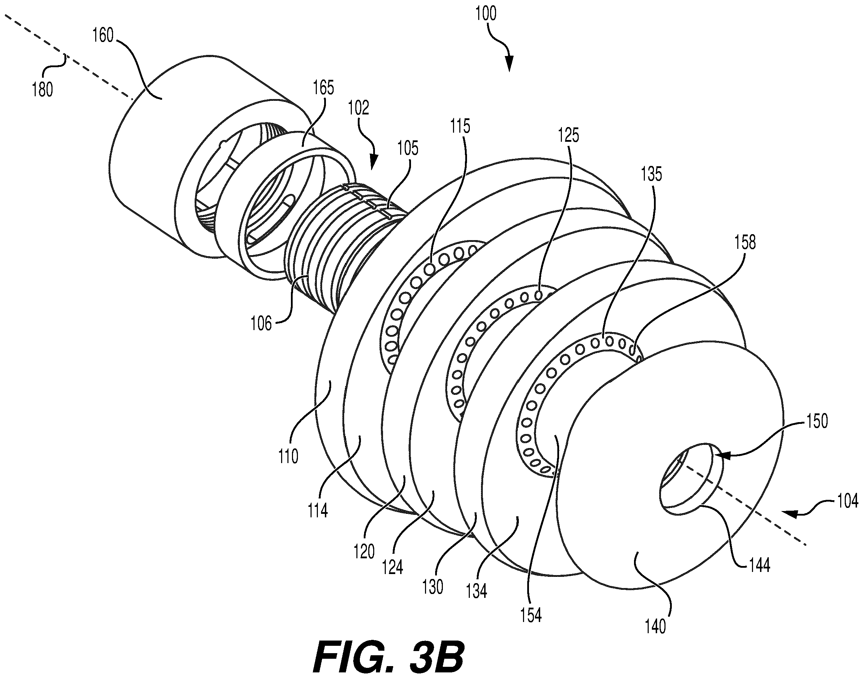

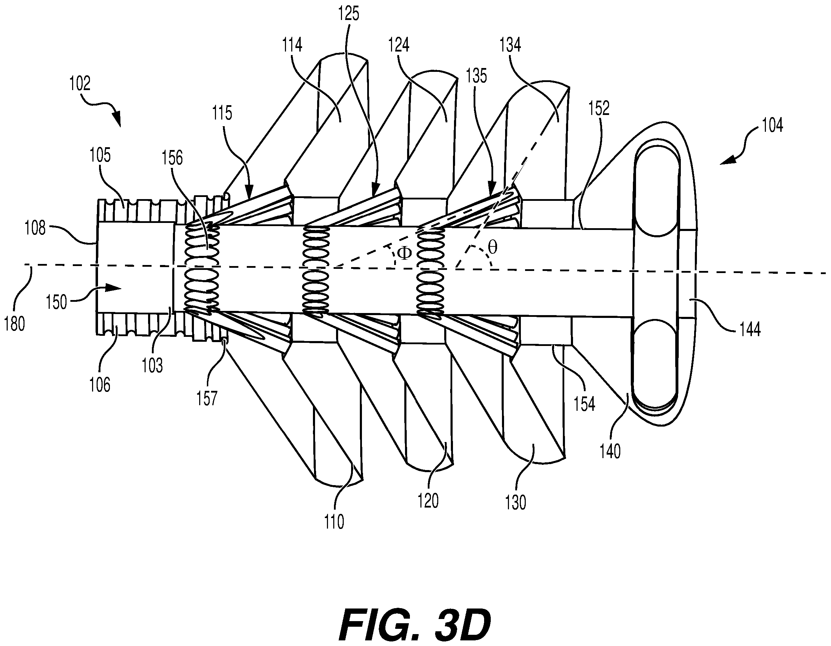

FIGs. 3A-3D depict the flow diverter 100, in accordance with the disclosed technology. As previously mentioned, and as will be described in greater detail herein, the flow diverter 100 can be configured to deliver a fluid radially about an end effector and/or to a target anatomical region. The flow diverter 100, can include a substantially tubular lumen 150 provided along a longitudinal axis 180 of the flow diverter 100, as shown in FIG. 3D.

The flow diverter 100 can comprise a plurality of manifolds 115, 125, 135. Each manifold 115, 125, 135 may comprise a plurality of fluid passageways 157 (as shown in FIG. 3D) extending from the lumen 150 to an exterior of the flow diverter 100. In some examples, each fluid passage 157 of the flow diverter 100 extends from an interior aperture 156 provided on an inner circumference 152 of the lumen 150 to an exterior aperture 158 provided about an outer circumference 154 of the lumen 150. In the example depicted in FIGs. 3A-3D, each manifold comprises 24 fluid passageways 157, however the number of fluid passageways may be varied. For example, each manifold may comprise 6, 12, 18, or 32 fluid passageways, or any number therebetween. In some examples, the manifolds of the flow diverter may comprise differing numbers of fluid passageways. For example, the number of fluid passageways may increase from the proximal end to the distal end of the flow diverter such that the second manifold 125 comprises more fluid passageways than the first manifold 115, and the third manifold 135 comprises more fluid passageways than the second manifold 125, or vice versa.

During operation, an end effector may be provided through the lumen 150 of the flow diverter 100. A distal aperture 144 provided at the distal end 104 of the flow diverter 100 may create a seal around the outer circumference of the end effector. As irrigation fluid is provided (e.g., by a pump) into a proximal aperture 108 provided at a proximal end 102 of the flow diverter 100, the irrigation fluid is forced through the fluid passageways 157 of the plurality of manifolds 115, 125, 135. In some examples, irrigation fluid also exits the distal aperture 144 of the flow diverter 100. In some examples, the seal created by the distal aperture 144 interfacing with the outer circumference of the end effector forces the irrigation fluid out only through the fluid passageways 157 of the plurality of manifolds 115, 125, 135. In some examples, the inner diameter of the distal aperture 144 is about 0.010 inches to about 0.050 inches. In some examples, the inner diameter of the distal aperture 144 is about 0.045 inches. In some examples, the inner diameter of the distal aperture 144 is about 0.255 millimeters (mm) to about 1.5 mm. In some examples, the inner diameter of the distal aperture 144 is about 1.17 mm.

In some examples, the flow diverter 100 comprises a plurality of annular ridges 110, 120, 130, 140. In some examples, the annular ridges can be provided to direct the irrigation fluid towards the end effector of the catheter. In some examples, the size of each annular ridge (i.e., circumference, radius, and diameter) decreases from the proximal annular ridge 110 towards the distal annular ridge 140, such that the annular ridges taper down from the proximal end 102 to the distal end 104 of the flow diverter. The annular ridges can be flexible such that they conform to the shape of the anatomical region or anatomical lumen which the flow diverter is provided in. In some examples, the flow diverter is formed from, or otherwise comprises, silicone. As depicted in FIGS. 3A-3D, the flow diverter 100 can comprise a proximal annular ridge 110, a second annular ridge 120, a third annular ridge 130, and distal annular ridge 140 arranged from the proximal end 102 to the distal end 104 along the longitudinal axis 180 of the flow diverter 100.

In some examples, each annular ridge comprises a proximal surface and a distal surface. For example, the proximal annular ridge 110 comprises a proximal surface 112 and a distal surface 114, the second annular ridge 120 comprises a proximal surface 122 and a distal surface 124, the third annular ridge 130 comprises a proximal surface 132 and a distal surface 134, and the distal annular ridge 140 comprises a proximal surface and a distal surface. In some examples, each annular ridge is substantially conical. In some examples, each of the proximal surfaces of the annular ridges can be substantially convex and each of the distal surfaces are substantially concave. In some examples, the distal surfaces 114, 124, 134 of the proximal annular ridge 110, the second annular ridge 120, and the third annular ridge 130 can be substantially concave and the distal surface of the distal annular ridge 140 can be substantially flat. In some examples, the distal surface of the distal annular ridge 140 can be substantially convex.

In some examples, the proximal surfaces (e.g., 112, 122, 132) of each of the annular ridges tapers toward the proximal end 102 of the flow diverter 100 such that the proximal surfaces are at an angle relative to the longitudinal axis 180 of the flow diverter 100. The angled proximal surfaces may facilitate removal of the flow diverter 100 from the target anatomical region and through the sheath 300.

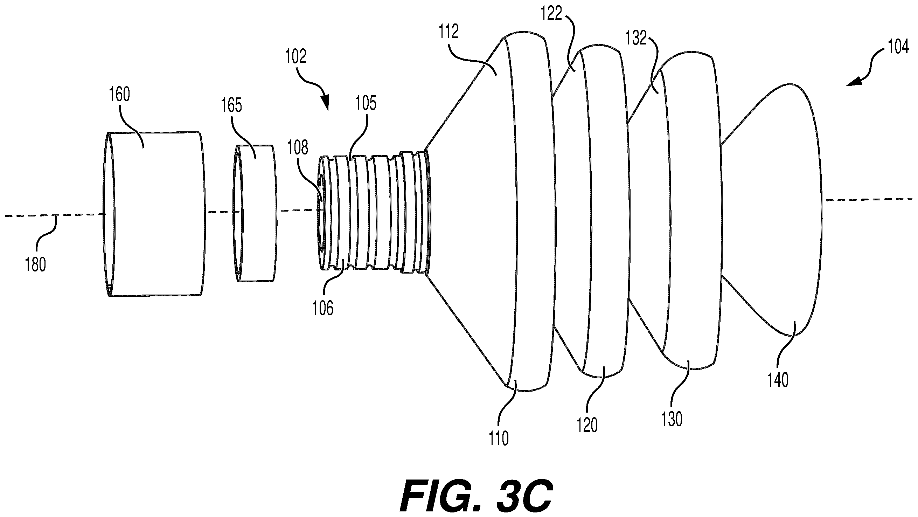

In some examples, the distal surfaces (e.g., 114, 124, 134) of each annular ridge are tapered toward the proximal end 102 of the flow diverter, which provides the distal surfaces at an angle relative to the longitudinal axis 180 of the flow diverter 100. The angle of the proximal surfaces and the distal surfaces may be approximately equal. In some examples, adjacent annular ridges are provided with some overlap. For example, spacing between the second annular ridge 120 and the third annular ridge 130 may be provided such that a portion of the distal surface 124 of the second annular ridge 120 overlaps a portion of the proximal surface 132 of the third annular ridge 130 when viewed from an angle transverse to the longitudinal axis 180 (as shown in FIG. 3C).

In some examples, each of the plurality of manifolds 115, 125, 135 can be provided between adjacent annular ridges. For example, exterior apertures 158 of the fluid passageways 157 of the first manifold 115 are provided between the proximal annular ridge 110 and the second annular ridge 120, exterior apertures 158 of the fluid passageways 157 of the second manifold 125 are provided between the second annular ridge 120 and the third annular ridge 130, and exterior apertures 158 of the fluid passageways 157 of the third manifold 135 are provided between the third annular ridge 130 and the distal annular ridge 140. In some examples, the exterior apertures 158 of the fluid passageways 157 of each manifold are provided on a distal surface of an annular ridge. For example, exterior apertures 158 of the fluid passageways 157 of the first manifold 115 are provided on the distal surface 114 of the proximal annular ridge 110, exterior apertures 158 of the fluid passageways 157 of the second manifold 125 are provided on the distal surface 124 of the second annular ridge 120, and exterior apertures 158 of the fluid passageways 157 of the third manifold 135 are provided on the distal surface 134 of the third annular ridge 130.

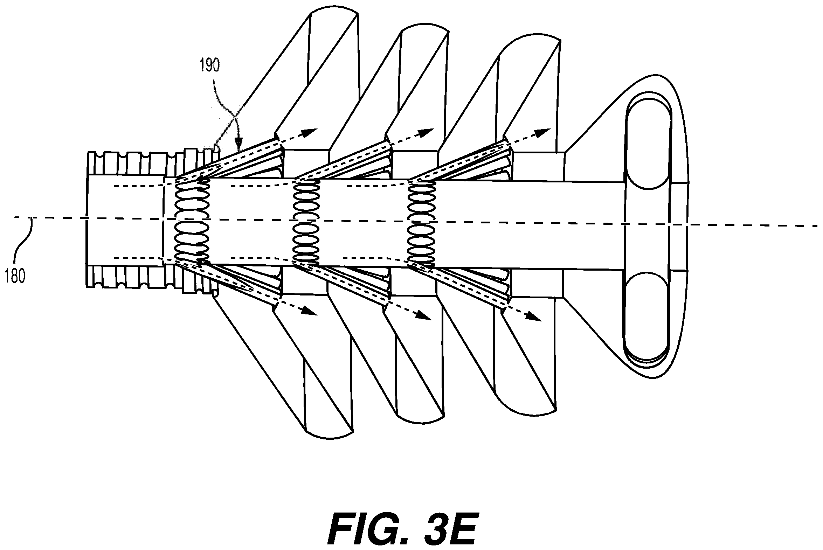

In some examples, the fluid passageways 157 extend outward from the lumen at an angle Φ away from the longitudinal axis. The angle Φ can be a predetermined angle sufficient to direct the irrigation fluid emitted from the fluid passageways of the manifolds and out the plurality of exterior apertures such that the fluid is directed generally radially about the longitudinal axis 180 (as depicted in FIG. 3E). As non-limiting examples, the angle Φ can be approximately 15°, 20°, 25°, 30°, 35°, 40°, 45°, 60°, 75°, 85°, or any other suitable angle for the particular application.

While the flow diverter 100 is exemplified in the figures with four annular ridges, other examples of the flow diverter 100 may include more or less annular ridges. For example, a flow diverter may include three, four, five, or six annular ridges. In some examples, the number of manifolds is one less than the number of annular ridges (i.e., if the number of annular ridges equals n, then the number of manifolds equals *n*-1). In some examples, the flow diverter 100 is formed by injection molding.

In some examples, the annular ridges extend outward from the lumen at an angle θ away from the longitudinal axis. The angle θ can be a predetermined angle sufficient to redirect the irrigation fluid emitted from the fluid passageways of the manifolds and out the plurality of exterior apertures such that the fluid is directed generally transverse to the longitudinal axis 180 (as depicted in FIG. 3E). In some examples, the angle θ can direct the fluid toward electrodes provided on an end effector. As non-limiting examples, the angle θ can be approximately 15°, 20°, 25°, 30°, 35°, 40°, 45°, 60°, 75°, 85°, or any other suitable angle for the particular application.

As depicted in FIG. 3D, the distal end of the lumen 150 may comprise a ledge 103. The ledge 103 may be provided to abut the distal end an insertion shaft (e.g., insertion shaft 350 as depicted in FIG. 2) of a catheter as the insertion shaft is received by the proximal aperture 108 and provide into the distal end of the lumen 150 of the flow diverter 100.

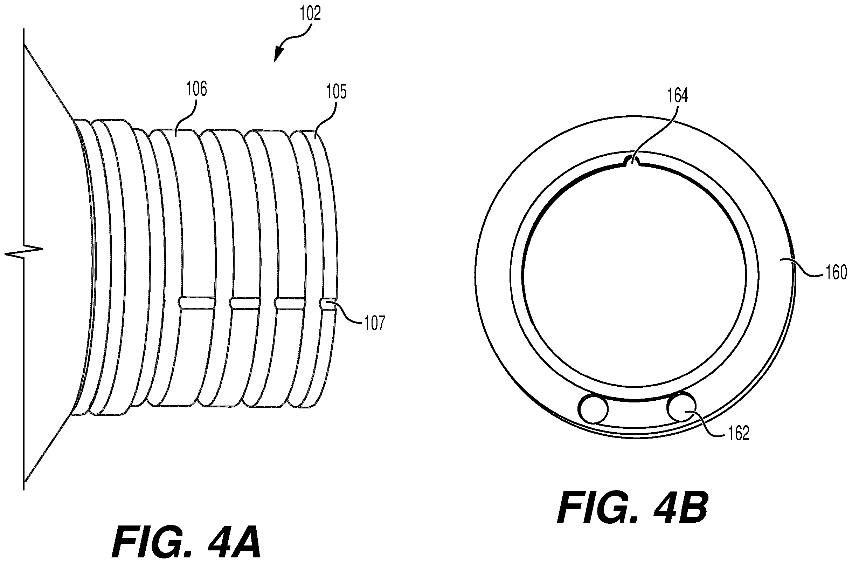

In some examples, a proximal end 102 of the flow diverter 100 comprises a corrugated tube 105. Corrugations 106 may be provided for attachment of the flow diverter 100 to a distal end of a sheath (e.g., sheath 300 as depicted in FIG. 2). Attachment of the flow diverter 100 to a distal end of a sheath may comprise placing the distal end of the sheath over the corrugated tube 105, then placing an electrode 165 over the outer circumference of the sheath and securing the sheath to the flow diverter by coupling a collar 160 over both the electrode 165 and the distal end of the sheath. The electrode 165 can be configured for non-contact intracardiac electrogram signal sensing. As depicted in FIG. 4A, the corrugated tube 105 provided at the proximal end 102 of the flow diverter may comprise a channel 107 provided through one or more of the corrugations. The channel 107 may align with a slot (e.g., slot 164 depicted in FIG. 4B) provided in the collar provide space for a lead wire to connect to the non-contact electrode (e.g., electrode 165 depicted in FIG. 3C).

FIG. 4B depicts the collar 160 component of the flow diverter 100. In some examples, the collar comprises a slot 164 which aligns with a channel (e.g., channel 107 as depicted in FIG. 4A) provided through the corrugations at the proximal end of the flow diverter to provide room for a lead wire to connect to the non-contact electrode (e.g., electrode 165 depicted in FIG. 3C). The collar 160 may further comprise holes 162 to accommodate lead wires. The lead wires may, for example, be connected to a therapeutic coil or electrode (not shown) provided near or underneath the electrode 165. The therapeutic coil or electrode, for example, can be configured for position sensing, delivering ablative energy, or detecting electrophysiological signals.

The disclosed technology described herein can be further understood according to the following clauses:
Clause 1: A flow diverter comprising: a proximal end and a distal end; a lumen extending along a longitudinal axis from the proximal end to the distal end of the flow diverter; a plurality of annular ridges disposed around the lumen between the proximal end and the distal end; and a plurality of manifolds, each manifold being disposed between adjacent annular ridges of the plurality of annular ridges and comprising a plurality of fluid passageways, each fluid passageway extending from the lumen to an exterior of the flow diverter.
Clause 2: The flow diverter of Clause 1, wherein the distal end comprises a distal aperture, the distal aperture configured to form a seal around an outer circumference of an end effector disposed through the lumen.
Clause 3: The flow diverter of Clause 1 or 2, wherein the plurality of annular ridges comprises a proximal annular ridge, a second annular ridge, a third annular ridge, and a distal annular ridge.
Clause 4: The flow diverter of Clause 3, wherein the plurality of manifolds comprises a first manifold disposed between the proximal annular ridge and the second annular ridge, a second manifold disposed between the second annular ridge and the third annular ridge, and a third manifold disposed between the third annular ridge and the distal annular ridge.
Clause 5: The flow diverter of Clause 4, wherein each of the proximal annular ridge, the second annular ridge, and the third annular ridge comprise a distal surface and a proximal surface, wherein each of the proximal surfaces are substantially convex and each of the distal surfaces are substantially concave.
Clause 6: The flow diverter of Clause 5, wherein the distal annular ridge comprises a distal surface and a proximal surface, wherein both the distal surface and the proximal surface of the distal annular ridge are substantially convex.
Clause 7: The flow diverter of Clause 3, wherein a diameter of the proximal annular ridge is greater than a diameter of the second annular ridge, the diameter of the second annular ridge is greater than a diameter of the third annular ridge, and the diameter of the third annular ridge is greater than a diameter of the distal annular ridge.
Clause 8: The flow diverter of any one of Clauses 1 to 7, wherein the flow diverter comprises an elastomeric material.
Clause 9: The flow diverter of Clause 8, wherein the flow diverter comprises silicone.
Clause 10: A catheter comprising: an insertion shaft extending along a longitudinal axis; an end effector disposed at a distal end of the insertion shaft; a sheath disposed around the insertion shaft; and a flow diverter disposed at a distal end of the sheath, the flow diverter comprising; a proximal end and a distal end; a lumen extending along the longitudinal axis from the proximal end to the distal end of the flow diverter; a plurality of annular ridges disposed around the lumen between the proximal end and the distal end; and a plurality of manifolds, each manifold being disposed between adjacent annular ridges of the plurality of annular ridges and comprising a plurality of fluid passageways, each fluid passageway extending from the lumen to an exterior of the flow diverter.
Clause 11: The catheter of Clause 10, wherein the plurality of manifolds diverts irrigation fluid from the lumen to the exterior of the flow diverter to distribute the irrigation fluid radially about the end effector.
Clause 12: The catheter of Clause 11, wherein the end effector comprises one or more electrodes configured for tissue ablation, and wherein the irrigation fluid cools the electrodes during ablation.
Clause 13: The catheter of Clause 11, wherein the end effector is a guidewire.
Clause 14: The catheter of any one of Clauses 10 to 13, wherein a diameter of each annular ridge of the plurality of annular ridges decreases relative to one another from the proximal end to the distal of the flow diverter.
Clause 15: The catheter of any one of Clauses 10 to 14, wherein the plurality of annular ridges comprises a proximal annular ridge, a second annular ridge, a third annular ridge, and a distal annular ridge.
Clause 16: The catheter of Clause 15, wherein the plurality of manifolds comprises a first manifold disposed between the proximal annular ridge and the second annular ridge, a second manifold disposed between the second annular ridge and the third annular ridge, and a third manifold disposed between the third annular ridge and the distal annular ridge.
Clause 17: The catheter of Clause 15 or 16, wherein each of the proximal annular ridge, the second annular ridge, and the third annular ridge comprise a distal surface and a proximal surface, wherein each of the proximal surfaces are substantially convex and each of the distal surfaces are substantially concave.
Clause 18: The catheter of any one of Clauses 10 to 17, wherein the distal end of the flow diverter comprises a distal aperture, the distal aperture forming a seal about an outer circumference of the end effector disposed through the lumen.
Clause 19. The catheter of Clause 18, wherein the seal comprises an elastomeric material.
Clause 20. The catheter of Clause 19, wherein the seal comprises silicone.

The embodiments described above are cited by way of example, and the present invention is not limited by what has been particularly shown and described hereinabove. Rather, the scope of the invention includes both combinations and sub combinations of the various features described hereinabove, as well as variations and modifications thereof which would occur to persons skilled in the art upon reading the foregoing description and which are not disclosed in the prior art.

## Claims

1. A flow diverter comprising:
a proximal end and a distal end;
a lumen extending along a longitudinal axis from the proximal end to the distal end of the flow diverter;
a plurality of annular ridges disposed around the lumen between the proximal end and the distal end; and
a plurality of manifolds, each manifold being disposed between adjacent annular ridges of the plurality of annular ridges and comprising a plurality of fluid passageways, each fluid passageway extending from the lumen to an exterior of the flow diverter.

2. The flow diverter of claim 1, wherein the distal end comprises a distal aperture, the distal aperture configured to form a seal around an outer circumference of an end effector disposed through the lumen.

3. A catheter comprising:
an insertion shaft extending along a longitudinal axis;
an end effector disposed at a distal end of the insertion shaft;
a sheath disposed around the insertion shaft; and
a flow diverter disposed at a distal end of the sheath, the flow diverter comprising;
a proximal end and a distal end;
a lumen extending along the longitudinal axis from the proximal end to the distal end of the flow diverter;
a plurality of annular ridges disposed around the lumen between the proximal end and the distal end; and
a plurality of manifolds, each manifold being disposed between adjacent annular ridges of the plurality of annular ridges and comprising a plurality of fluid passageways, each fluid passageway extending from the lumen to an exterior of the flow diverter.

4. The catheter of claim 3, wherein the plurality of manifolds diverts irrigation fluid from the lumen to the exterior of the flow diverter to distribute the irrigation fluid radially about the end effector.

5. The catheter of claim 4, wherein the end effector comprises one or more electrodes configured for tissue ablation, and wherein the irrigation fluid cools the electrodes during ablation.

6. The catheter of claim 4, wherein the end effector is a guidewire.

7. The catheter of any of claims 3 to 6, wherein a diameter of each annular ridge of the plurality of annular ridges decreases relative to one another from the proximal end to the distal of the flow diverter.

8. The flow diverter of claim 1 or claim 2, or the catheter of any of claims 10 to 14, wherein the plurality of annular ridges comprises a proximal annular ridge, a second annular ridge, a third annular ridge, and a distal annular ridge.

9. The flow diverter or the catheter of claim 8, wherein the plurality of manifolds comprises a first manifold disposed between the proximal annular ridge and the second annular ridge, a second manifold disposed between the second annular ridge and the third annular ridge, and a third manifold disposed between the third annular ridge and the distal annular ridge.

10. The flow diverter of claim 9 or the catheter of claim 8 or claim 9, wherein each of the proximal annular ridge, the second annular ridge, and the third annular ridge comprise a distal surface and a proximal surface, wherein each of the proximal surfaces are substantially convex and each of the distal surfaces are substantially concave.

11. The catheter of any of claims 4 to 10, wherein the distal end of the flow diverter comprises a distal aperture, the distal aperture forming a seal about an outer circumference of the end effector disposed through the lumen.

12. The flow diverter of any of claims 1, 2 or 8 to 10 when dependent upon 1 or 2, or the catheter of claim 18, wherein the seal comprises an elastomeric material.

13. The flow diverter or the catheter of claim 12, wherein the seal comprises silicone.

14. The flow diverter of claim 10, wherein the distal annular ridge comprises a distal surface and a proximal surface, wherein both the distal surface and the proximal surface of the distal annular ridge are substantially convex.

15. The flow diverter of claim 8, wherein a diameter of the proximal annular ridge is greater than a diameter of the second annular ridge, the diameter of the second annular ridge is greater than a diameter of the third annular ridge, and the diameter of the third annular ridge is greater than a diameter of the distal annular ridge.
